# EUROPÄISCHE PATENTSCHRIFT

(11) **EP 3 084 364 B1**
(45) Veröffentlichungstag und Bekanntmachung des Hinweises auf die Patenterteilung: **29.07.2020**
(21) Anmeldenummer: 14793146.3
(22) Anmeldetag: 04.11.2014
(51) Int. Cl.: G01F 1/696, G01F 15/02, G01F 15/04, G01K 13/02, G01F 1/684

(54) **LUFTMASSENMESSVORRICHTUNG, LUFTMASSENMESSSYSTEM UND LUFTMASSENMESSVERFAHREN FÜR EIN FAHRZEUG**
AIR MASS MEASURING APPARATUS, AIR MASS MEASURING SYSTEM AND AIR MASS MEASURING METHOD FOR A VEHICLE
DISPOSITIF DE MESURE DE MASSES D'AIR, SYSTÈME DE MESURE DE MASSES D'AIR, ET PROCÉDÉ DE MESURE DE MASSES D'AIR POUR UN VÉHICULE

(30) Priorität: 17.12.2013 DE 102013226140
(43) Veröffentlichungstag der Anmeldung: 26.10.2016
(73) Patentinhaber: Robert Bosch GmbH, 70442 Stuttgart (DE)
(72) Erfinder: SCHNEIDER, Norbert, 75233 Tiefenbronn (DE); HORSTBRINK, Michael, 70469 Stuttgart-Feuerbach (DE); KONZELMANN, Uwe, 71679 Asperg (DE); KUEHN, Andreas, 76275 Ettlingen (DE); RITTMANN, Michael, 71254 Ditzingen (DE)
(86) Internationale Anmeldenummer: PCT/EP2014/073697
(87) Internationale Veröffentlichungsnummer: WO 2015/090713

(56) Entgegenhaltungen:
- EP-A1- 1 637 847
- DE-A1- 19 750 496
- DE-A1-102010 043 062

## Beschreibung

### Stand der Technik

Die vorliegende Erfindung bezieht sich auf eine Luftmassenmessvorrichtung, ein Luftmassenmesssystem und ein Luftmassenmessverfahren für ein Fahrzeug, die beispielsweise zur Luftmassenmessung bei einem Verbrennungsmotor eines Fahrzeugs eingesetzt werden können.

Die Luftmassenmessung ist bei Benzin- und Dieselmotoren etabliert. Sie dient zur Regelung der Kraftstoff-Einspritzmenge und der Abgasrückführung.

Die EP 1 637 847 A1 offenbart eine Luftmassenmessvorrichtung mit einer Ausgangsschnittstelle mit einem Versorgungsspannungsanschluss, einem Anschluss zum Ausgeben eines Lufttemperatursignals, einem Anschluss zum Ausgeben eines Wandtemperatursignals sowie einem Anschluss zum Ausgeben eines Luftflussratensignals.

Die DE 197 50 496 A1 offenbart einen Sensor, umfassend einen Luftmassenmesser mit einem Sensorelement zur Erfassung einer angesaugten Luftmasse, benachbart zu dem ein Feuchtesensor und/oder ein Drucksensor sowie eine Auswerteschaltung zur Verarbeitung der von dem Luftmassenmesser, dem Feuchtesensor und dem Drucksensor ausgegebenen Daten vorgesehen und in einem einzigen Gehäuse angeordnet sind.

Die DE 10 2010 043 062 A1 offenbart eine Sensorvorrichtung zur Erfassung einer Strömungseigenschaft eines fluiden Mediums.

### Offenbarung der Erfindung

Vor diesem Hintergrund werden mit der vorliegenden Erfindung eine Luftmassenmessvorrichtung, ein Luftmassenmesssystem und ein Luftmassenmessverfahren für ein Fahrzeug gemäß den Hauptansprüchen vorgestellt. Vorteilhafte Ausgestaltungen ergeben sich aus den jeweiligen Unteransprüchen und der nachfolgenden Beschreibung.

Vorteilhafterweise kann von einer Luftmassenmessvorrichtung neben der Luftmasse zumindest eine physikalische Größe erfasst werden. Die erfassten Daten können gebündelt über einen Anschluss weitergeleitet werden. Im Vergleich zu einer ungebündelten Weiterleitung kann eine Anzahl der zum Weiterleiten der erfassten Daten erforderlichen Leitungen verringert werden. Auch wenn in den Ausführungsformen und Ausführungsbeispielen von Luft gesprochen wird, kann die Luftmassenmessvorrichtung zur Messung der Masse eines beliebigen Gases oder Gasgemisches eingesetzt werden. Auch ist die Anwendung im Fahrzeugbereich lediglich beispielhaft gewählt.

Eine Luftmassenmessvorrichtung für ein Fahrzeug weist die folgenden Merkmale auf:
ein Trägerelement;
einen Luftmassensensor zum Bereitstellen von Luftmassendaten, wobei der der Luftmassensensor an dem Trägerelement angeordnet ist;
zumindest einen weiteren Sensor zum Bereitstellen weiterer Sensordaten, wobei der zumindest eine weitere Sensor an dem Trägerelement angeordnet ist; und
eine Auswerteschaltung mit einer ersten Eingangsschnittstelle zum Empfangen der Luftmassendaten, zumindest einer zweiten Eingansschnittstelle zum Empfangen der weiteren Sensordaten und mit einer Ausgangsschnittstelle, wobei die Auswerteschaltung an dem Trägerelement angeordnet und ausgebildet ist, um die Luftmassendaten und die weiteren Sensordaten als gebündelte Sensordaten über die Ausgangsschnittstelle bereitzustellen.

Die Luftmassenmessvorrichtung kann beispielsweise zum Ermitteln einer einem Verbrennungsmotor eines Fahrzeugs zugeführten Luftmasse verwendet werden. Beispielsweise kann die Luftmassenmessvorrichtung als ein sogenannter Integrated Pressure- and Humidity Sensor ausgeführt sein. Die von der Auswerteschaltung an der Ausgangsschnittstelle bereitgestellten Daten können beispielsweise von einem Steuergerät des Fahrzeugs empfangen und weiterverarbeitet werden. Das Trägerelement kann ausgeformt sein, um in einer Elektronikkammer eines Gehäuses angeordnet zu werden. Der weitere Sensor kann ausgebildet sein, um einen weiteren physikalischen Parameter der Luft, deren Masse von dem Luftmassensensor erfasst wird, zu erfassen. Die Auswerteschaltung kann als eine integrierte Schaltung ausgeführt sein. Der zumindest eine Sensor kann Signalaufbereitungseinrichtung zum Aufbereiten von erfassten Sensorwerten umfassen und ausgebildet sein, um die Sensordaten als aufbereitete Daten an die Auswerteschaltung zu übermitteln. Die Auswerteschaltung kann ausgebildet sein, um die über die Empfangsschnittstellen empfangenen Daten entsprechend eines Übertragungsprotokolls der Ausgangsschnittstelle zu den gebündelten Sensordaten zu bündeln. Beispielsweise können die gebündelten Daten zeitgleich oder zeitlich versetzt an der Ausgangsschnittstelle bereitgestellt werden.

Gemäß einer Ausführungsform kann die Luftmassenmessvorrichtung als den zumindest einen weiteren Sensor einen Luftfeuchtesensor und zusätzlich oder alternativ einen Drucksensor aufweisen. Auf diese Weise können eine Luftfeuchte sowie ein Druck der Luft zusammen mit der Masse der Luft erfasst werden.

Zusätzlich oder alternativ kann die Luftmassenmessvorrichtung einen Temperatursensor aufweisen. Der Temperatursensor kann über eine Sensorschnittstelle des Trägerelements mit dem Trägerelement verbunden sein. So kann ein Temperaturfühler des Temperatursensors beispielsweise benachbart zu dem des Trägerelements angeordnet sein. Somit kann zusätzlich zu der Masse auch die Temperatur der Luft erfasst werden.

Die Auswerteschaltung kann die zweite Eingansschnittstelle zum Empfangen von einem Luftfeuchtesensor bereitgestellten Luftfeuchtedaten, eine dritte Eingansschnittstelle zum Empfangen von einem Drucksensor bereitgestellten Druckdaten und eine vierte Eingansschnittstelle zum Empfangen von einem Temperatursensor bereitgestellten Temperaturdaten aufweisen. Die Auswerteschaltung kann ausgebildet sein, um die Luftmassendaten, die Luftfeuchtedaten, die Druckdaten und die Temperaturdaten gebündelt über die Ausgangsschnittstelle bereitzustellen. Dabei ist es nicht erforderlich, dass alle hier genannten Sensoren bestückt sind. Auf diese Weise ist eine große Flexibilität gegeben. Es sind beispielsweise unterschiedliche Funktionsvarianten mit zusätzlicher Temperatur-, Druck- oder Feuchtemessung realisierbar.

Die Ausgangsschnittstelle der Auswerteschaltung kann dreiadrig ausgeführt sein. Über die Anschlüsse der Ausgangsschnittstelle kann die Auswerteschaltung beispielsweise mit einer Versorgungsspannung und mit Masse verbunden werden. Über den dritten Anschluss kann eine Signalspannung zur Darstellung der gebündelten Sensordaten übertragen werden. Beispielsweise kann die Signalspannung ein amplituden- oder frequenzmoduliertes Signal darstellen. Eine solche Ausgangsschnittstelle kann beispielsweise als SENT-Schnittstelle (Single Edge Nibble Transmission) ausgeführt sein. Die Ausgangsschnittstelle kann als eine digitale Schnittstelle ausgeführt sein, die eine digitale Signalübertragung beispielsweise zu einem Steuergerät ermöglicht.

Das Trägerelement kann einen Sensorträger und eine Leiterplatte aufweisen. Der Luftmassensensor kann an dem Sensorträger angeordnet sein. Der zumindest eine weitere Sensor und die Auswerteschaltung können an der Leiterplatte angeordnet sein. Der Luftmassensensor oder der Sensorträger können über elektrische Leitungen mit der Auswerteschaltung verbunden sein. Das Elektronikmodul kann ein Grundelement aufweisen, an dem der Sensorträger und die Leiterplatte befestigt sind. Ein solches Grundelement kann beispielsweise ein Blech sein. Das Grundelement, der Sensorträger und die Leiterplatte können starr miteinander verbunden sein. Das Grundelement kann verwendet werden, um das Elektronikmodul an einem Gehäuse zu befestigen. Dazu kann das Elektronikmodul eine Befestigungsschnittstelle zum Befestigen des Elektronikmoduls an einem Gehäuse aufweisen.

Die Auswerteschaltung und der zumindest eine weitere Sensor können auf derselben Seite der Leiterplatte angeordnet sein. Dies erleichtert das Bestücken der Leiterplatte.

Entsprechend kann die Luftmassenmessvorrichtung ein Gehäuse umfassen. Das Gehäuse kann eine Elektronikkammer, einen Messkanal zum Leiten eines Gases und einen elektrischen Anschluss aufweisen. Das Elektronikmodul kann in der Elektronikkammer angeordnet sein. Der Luftmassensensor kann in den Messkanal eingeführt angeordnet sein. Die Ausgangsschnittstelle der Auswerteschaltung kann über elektrische Leitungen mit dem elektrischen Anschluss verbunden sein, um die gebündelten Sensordaten an den elektrischen Anschluss bereitzustellen. Der Anschluss kann eine externe Schnittstelle der Luftmassenmessvorrichtung darstellen. Das Gehäuse kann beispielsweise als ein Steckfühlergehäuse ausgeführt sein. Durch den Messkanal kann das Gas, beispielsweise Luft, dessen Masse zu bestimmen ist, geleitet werden. Auf diese Weise kann die Luftmassenmessvorrichtung als eine kompakte Einheit darstellen.

Ein Luftmassenmesssystem für ein Fahrzeug weist eine in einem Ansaugtrakt eines Verbrennungsmotors eines Fahrzeugs angeordnete oder anordenbare Luftmassenmessvorrichtung auf. Dabei ist die Luftmassenmessvorrichtung über den elektrischen Anschluss mit einem Steuergerät des Fahrzeugs verbunden oder verbindbar. Somit eignet sich die genannte Luftmassenmessvorrichtung vorteilhaft zum Einsatz in einem Kraftfahrzeug, beispielsweise einem Personenkraftwagen oder einem Lastkraftwagen.

Ein Luftmassenmessverfahren für ein Fahrzeug umfasst die folgenden Schritte:
Empfangen von Luftmassendaten über eine erste Eingangsschnittstelle einer an einem Trägerelement angeordneten Auswerteschaltung, wobei die Luftmassendaten von einem an dem Trägerelement angeordneten Luftmassensensor bereitgestellte Daten repräsentieren;
Empfangen von weiteren Sensordaten über zumindest eine zweite Eingangsschnittstelle der Auswerteschaltung, wobei die weiteren Sensordaten von zumindest einem an dem Trägerelement angeordneten weiteren Sensor bereitgestellte Daten repräsentieren; und
Bereitstellen der Luftmassendaten und der weiteren Sensordaten als gebündelte Sensordaten über eine Ausgangsschnittstelle der Auswerteschaltung.

Die genannten Schritte können vorteilhaft unter Verwendung von Einrichtungen der genannten Luftmassenmessvorrichtung umgesetzt werden.

Die Erfindung wird durch die Ansprüche definiert und nachstehend anhand der beigefügten Zeichnungen beispielhaft näher erläutert. Es zeigen:
Fig. 1 eine schematische Darstellung eines Fahrzeugs mit einer Luftmassenmessvorrichtung;
Fig. 2 eine schematische Darstellung einer Luftmassenmessvorrichtung;
Fig. 3 eine Darstellung von Einrichtungen einer Luftmassenmessvorrichtung;
Fig. 4 ein Blockschaltbild einer Luftmassenmessvorrichtung; und
Fig. 5 ein Ablaufdiagramm eines Luftmassenmessverfahrens.

In der nachfolgenden Beschreibung bevorzugter Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

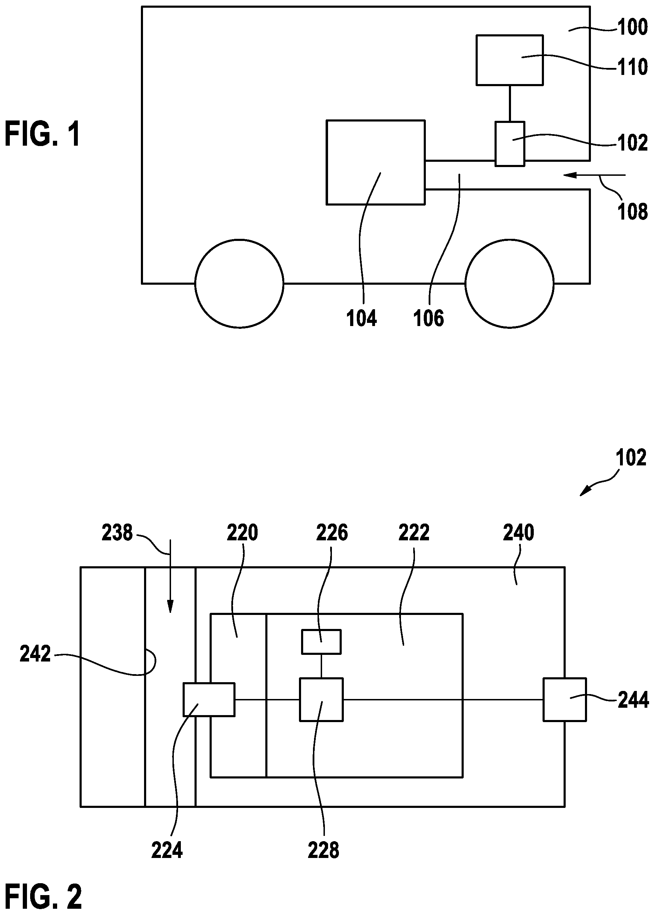

Fig. 1 zeigt eine schematische Darstellung eines Fahrzeugs 100 mit einer Luftmassenmessvorrichtung 102 gemäß einem Ausführungsbeispiel der vorliegenden Erfindung. Das Fahrzeug 100 weist einen Verbrennungsmotor 104 auf, dem über einen Luftkanal 106 Luft 108 zugeführt wird. Die Luftmassenmessvorrichtung 102 ist in oder an dem Luftkanal 106 angeordnet und ausgebildet, um einen eine Luftmasse der Luft 108 repräsentierenden Wert zu erfassen und gemäß diesem Ausführungsbeispiel an ein Steuergerät 110 des Fahrzeugs 100 bereitzustellen.

Gemäß unterschiedlicher Ausführungsbeispiele kann die Luftmassenmessvorrichtung 102 ferner ausgebildet sein, um eine Temperatur, einen Druck und/oder eine Luftfeuchtigkeit der Luft 108 repräsentierende Werte zu erfassen und als gemeinsame Daten zusammen mit dem die Luftmasse repräsentierenden Wert bereitzustellen.

Fig. 2 zeigt eine schematische Darstellung einer Luftmassenmessvorrichtung 102 gemäß einem Ausführungsbeispiel der vorliegenden Erfindung. Die Luftmassenmessvorrichtung 102 weist ein Trägerelement auf. Das Trägerelement kann einstückig ausgeführt sein, oder wie in Fig. 2 gezeigt, einen Sensorträger 220 und eine Leiterplatte 222 umfassen, die mechanisch miteinander verbunden sind.

Ein Luftmassensensor 224 ist an dem Sensorträger 220 angeordnet. An der Leiterplatte 222 sind zumindest ein weiterer Sensor 226 und eine Auswerteschaltung 228 angeordnet. Der weiterere Sensor 226 und die Auswerteschaltung 228 sind gemäß diesem Ausführungsbeispiel auf derselben Oberseite der Leiterplatte 222 angeordnet.

Der Luftmassensensor 224 ist ausgebildet, um Luftmassendaten bezüglich eines Luftmassenstroms 238 bereitzustellen. Der Luftmassensensor 224 ist ausgebildet, um die Luftmassendaten über elektrische Leitungen an die Auswerteschaltung 228 bereitzustellen.

Der zumindest eine weitere Sensor 226 ist ausgebildet, weiterer Sensordaten bezüglich des Luftmassenstroms 236, beispielsweise eine Temperatur, eine Luftfeuchtigkeit oder einen Druck des Luftmassenstroms 236 zu erfassen und über elektrische Leitungen an die Auswerteschaltung 228 bereitzustellen.

Die Auswerteschaltung 228 weist gemäß diesem Ausführungsbeispiel für jeden Sensor 224, 226 eine Eingangsschnittstelle zum Empfangen der jeweiligen Sensordaten der Sensoren 224, 226 auf. Ferner weist die Auswerteschaltung 228 eine Ausgangsschnittstelle auf. Die Auswerteschaltung 228 ist ausgebildet, um die Luftmassendaten des Luftmassensensors 224 und die weiteren Sensordaten des zumindest einen weiteren Sensors 226 über separate Schnittstellen zu empfangen und als gebündelte Sensordaten über die gemeinsame Ausgangsschnittstelle bereitzustellen. Beispielsweise kann die Ausgangsschnittstelle eine Leitung oder ein Leitungspaar aufweisen, über die sowohl die Luftmassendaten repräsentierende Signale als auch die weiteren Sensordaten repräsentierende Signale übertragen werden können. Dazu kann die Auswerteschaltung 228 ausgebildet sein, um die Luftmassendaten und die weiteren Sensordaten entsprechend einem Übertragungsprotokoll der Ausgangsschnittstelle miteinander zu kombinieren.

Die Luftmassenmessvorrichtung 102 kann gemäß einem Ausführungsbeispiel als ein Elektronikmodul aufgefasst werden, das in einem Gehäuse 240 angeordnet werden kann.

Alternativ kann die Luftmassenmessvorrichtung 102, wie in Fig. 2 gezeigt, als ein Modul aufgefasst werden, das das Gehäuse 240 mit einer Elektronikkammer aufweist, in der der Sensorträger 220 mit dem Luftmassensensor 224 und die Leiterplatte 222 mit dem weiteren Sensor 226 und der Auswerteschaltung 228 angeordnet sind. Das Gehäuse 240 weist einen Messkanal 242 zum Leiten des Massenstroms 238 auf. Der Luftmassensensor 224 kann in den Messkanal 242 eingeführt angeordnet sein. Das Gehäuse 240 weist als externe Schnittstelle, über die der Luftmassenmessvorrichtung 102 beispielsweise mit einem Steuergerät verbunden werden kann, einen elektrischen Anschluss 244 auf. Der elektrische Anschluss 244 ist mit der Ausgangsschnittstelle der Auswerteschaltung 228 über elektrische Leitungen verbunden. Ein Übertragungsprotokoll von über den elektrischen Anschluss 244 geleiteten Daten kann einem Übertragungsprotokoll der Ausgangsschnittstelle entsprechen.

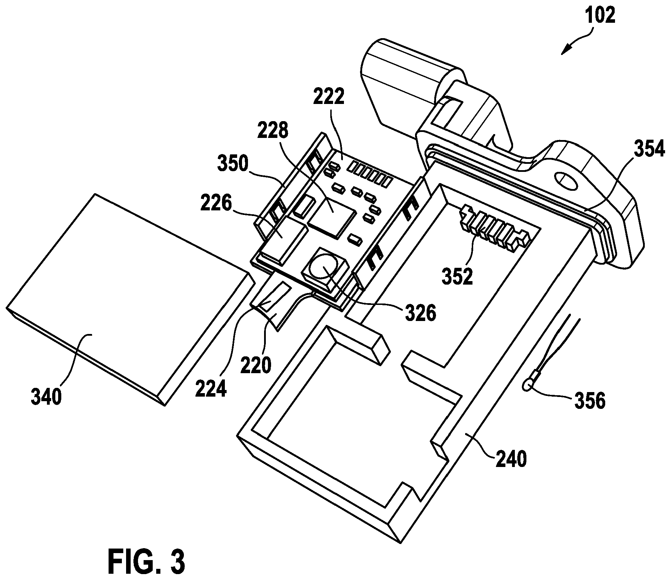

Fig. 3 zeigt eine Darstellung von Einrichtungen einer Luftmassenmessvorrichtung 102 gemäß einem Ausführungsbeispiel der vorliegenden Erfindung. Die Luftmessvorrichtung 102 ist gemäß diesem Ausführungsbeispiel als ein Steckfühler, insbesondere als ein Druck-, Feuchtigkeits- und Temperatur-Steckfühler, ausgeführt.

Gezeigt ist ein Elektronikmodul, das einen Sensorträger 220 für einen Luftmassensensor und eine Leiterplatte 222 mit einer Auswerteschaltung 228 und gemäß diesem Ausführungsbeispiel zwei weiteren Sensoren 226, 326 aufweist. Die Auswerteschaltung 228 ist als ein Auswerte-IC ausgeführt. Beispielhaft sind der weitere Sensor 226 als ein Drucksensor, beispielsweise ein lötbarer Drucksensor und der weitere Sensor 326 als ein Feuchtesensor, der beispielsweise eine semi-permeable Membran mit einem Messkammer-Feuchtesensor und einen Feuchtesensor umfasst, ausgeführt. Das Elektronikmodul umfasst ferner ein Bodenblech 350. Eine den Sensoren 226, 326 und der Auswerteschaltung 328 abgewandte Seite der Leiterplatte 222 liegt auf einem Boden des Bodenblechs 350 auf. Das Bodenblech 350 weist zwei gegenüberliegende Wandbereiche auf, die rechtwinklig zu dem Boden des Bodenblechs 350 angeordnet sind und entlang zweier gegenüberliegender Ränder der Leiterplatte 222 geführt sind. Die Wandbereiche des Bodenblechs 350 weisen Befestigungsmittel auf, beispielsweise Durchgangslöcher und/oder Rasthaken, zum Befestigen des Bodenblechs 350 an einem Gehäuse 240 der Luftmassenmessvorrichtung 102. Der Sensorträger 220 ragt über ein freies Ende der Leiterplatte 222 hinaus, das von keinem Wandbereich des Bodenblechs 350 abgeschirmt ist. Der Sensorträger 220 ist in etwa mittig zu dem freien Ende der Leiterplatte 222 angeordnet. Der Sensorträger 220 ist in etwa halb so breit wie die Leiterplatte 222. Hauptoberflächen des Sensorträgers 220 und der Leiterplatte sind im Wesentlichen parallel zueinander ausgerichtet.

Das Gehäuse 240 weist eine Elektronikkammer zum Aufnehmen des Elektronikmoduls auf. Angrenzend an die Elektronikkammer weist das Gehäuse 240 einen Messkanal auf. Der Messkanal wird durch einen Messkanalabschnitt des Gehäuses 240 und durch ein weiteres Gehäuseteil 340 ausgeformt, das als ein Messkanal-(Bypass-) Deckel auf den Messkanalabschnitt des Gehäuses 240 aufgesetzt wird. Der Messkanal weist eine Mehrzahl von Biegungen zum Führen eines Gases durch das Gehäuse der Luftmassenmessvorrichtung 102 auf. Wenn das Elektronikmodul in der Elektronikkammer angeordnet ist, ragt der Sensorträger 220 in den Messkanal hinein. Auf diese Weise kann der an dem Sensorträger 220 angeordnete Luftmassensensor in direktem Kontakt mit durch den Messkanal geleiteten Gas stehen.

Die Elektronikkammer kann durch einen weiteren Deckel abgedeckt werden. Dabei kann es sich um einen Elektronikraumdeckel handeln. Durch den Elektronikraumdeckel kann eine Anbindung von Drucksensor 326 und Feuchtesensor 356 gegenüber der Umgebung abgedeckt werden.

Das Gehäuse 240 weist einen Leiterkamm 352 auf. Der Leiterkamm 352 weist eine Mehrzahl von elektrischen Leiterbahnen auf. Über den Leiterkamm 352 kann die Leiterplatte 222 und insbesondere die Auswerteschaltung 228 mit einem an dem Gehäuse 240 angeordneten elektrischen Anschluss der Luftmassenmessvorrichtung 102 verbunden werden.

An dem Gehäuse 240 kann ein umlaufender Dichtring 354 angeordnet sein. Wird die Luftmassenmessvorrichtung 102 beispielsweise mit dem den Messkanal aufweisenden Abschnitt des Gehäuses 240 voran in einen Luftkanal 106 eingeführt, so kann eine Außenwand des Luftkanals, durch den die Luftmassenmessvorrichtung 102 geführt ist, durch den Dichtring 354 abgedichtet werden. Der Anschluss der Luftmassenmessvorrichtung 102 kann dabei außerhalb des Luftkanals angeordnet sein.

Gemäß diesem Ausführungsbeispiel weist die Luftmassenmessvorrichtung 102 einen weiteren Sensor in Form eines Temperaturfühlers 356 auf. Der Temperaturfühler 356 kann beispielsweise benachbart zu der Leiterplatte 222 angeordnet werden. Anschlüsse des Temperaturfühlers 356 können über den Leiterkamm 352 kontaktiert werden.

Die Sensoren 226, 326, 356 können als Sensorsatelliten bezeichnet werden.

Gemäß einem Ausführungsbeispiel ist die Luftmassenmessvorrichtung 102 als ein Luftmassensensor 102 mit einer Integration von Druck- und Feuchtesensor 226, 326 in einem Steckfühler mit digitaler SENT-Schnittstelle ausgeführt.

Bei dem Luftmassensensor 102 (Integrated Pressure- and Humidity Sensor) werden neben Luftmasse und Ansauglufttemperatur weitere Messgrößen wie Druck, relative Feuchte und Temperatur am Feuchtesensor 326 ermittelt. Die einzelnen Sensoren 226, 326, 356 für Druck, Luftmasse, Feuchte und Temperatur arbeiten unabhängig voneinander. Die jeweiligen Messgrößen werden jeweils über eine separate Leitung an die Auswerteschaltung 228 übermittelt. Aufgrund der gemeinsamen Ausgangsschnittstelle der Auswerteschaltung 228 ist an dem Gehäuse 240 des Luftmassensensors 102 kein 8-poliger Stecker notwendig, sondern beispielsweise ein 3-poliger Stecker als Anschluss ausreichend. Ferner sind auch keine zwei Elektronikkammern auf Vorder- und Rückseite des Steckfühlers erforderlich, sondern es ist eine einzige Elektronikkammer ausreichend. In der gemeinsamen Elektronikkammer befindet sich sowohl die Elektronik für die Luftmassenmessung als auch die Elektronik für Druck- und Feuchtemessung. Die Elektronik für die Luftmassenmessung und auch die Montage von Drucksensor 226 und Feuchtemodul 356 kann auf einer Fertigungslinie erfolgen.

Der Luftmassensensor kann beispielsweise als ein mikromechischer-Sensor ausgeführt sein.

Der beschriebene Ansatz ermöglicht eine flexible, kostengünstige Integration von Druck- und Feuchtesensorik 226, 326 mit nur einer Auswerteschaltung 228 zu realisieren und die Messdaten über eine Signalleitung, beispielsweise an das Steuergerät zu übermitteln.

Durch die Integration von Druck- und Feuchtesensor 226, 326 auf dem Elektronikmodul des Luftmassenmessers 102 kann die bisher für die Montage des Drucksensors 226 und des Feuchtemoduls 326 notwendige Fertigungslinie komplett entfallen. Gleichzeitig kann die Anzahl der notwendigen Bauteile deutlich reduziert werden. Dies betrifft beispielsweise den Entfall einer weiteren Leiterplatte für das Feuchtemodul 526 oder eines Mikrocontrollers. Der Entfall einer zweiten Elektronikkammer macht den Einsatz eines kleineren Steckfühlergehäuses 240 mit verringerten Außenabmaßen und verbesserter Funktion, z. B. hinsichtlich eines Druckabfalls, möglich.

Durch die Anbindung der Sensor-Satelliten 226, 326, 356 an den Auswerte-IC 228 des Luftmassenmessers (HFM) 102 können alle Sensorsignale über eine Signalleitung zum Steuergerät gesendet werden. Dadurch lassen sich die Kosten, beispielsweise für einen Kabelbaum eines Fahrzeugs, durch die Verringerung der Anzahl der Steckerpins des Anschlusses des Luftmassenmessers 102 beispielsweise von 8 auf 3 deutlich reduzieren.

Der Luftmassenmesser 102 (Pressure, Temperature, Humidity) besitzt eine Elektronikkammer. Die Elektronikkammer dient der Aufnahme des Elektronikmoduls. Das Elektronikmodul besteht aus dem Bodenblech 350 mit angespritztem Sensorträger 220. Der Sensorträger 220 dient der Aufnahme des Sensors zur Luftmassenmessung. Auf dem Bodenblech 350 wird eine bestückte Leiterplatte 222 montiert. Die Verbindung der Leiterplatte 222 mit dem

Sensor zur Luftmassenmessung erfolgt gemäß einem Ausführungsbeispiel mittels Dünndrahtbonds. Alle elektrischen Bauelemente 226, 228, 326 mit Ausnahme des Temperaturfühlers 356 werden durch einen Standard-SMD Prozess auf der Leiterplatte 222 bestückt und gelötet. Die Bestückung erfolgt im Nutzen, d.h. auf mehreren Leiterplatten 222 gleichzeitig. Durch den SMD-Bestückungsprozess können relativ einfach und kostengünstig Varianten, z. B. ohne Drucksensor 226, dargestellt werden.

Der Feuchtesensor 326 sitzt in einer sogenannten Messkammer, deren Öffnung mit einer semi-permeablen Membran verschlossen ist. Diese ist für Feuchte durchlässig und schützt den Feuchtesensor 326 gleichzeitig aber vor eindringendem Wasser und Schmutz.

Druck- und Feuchtesensor 226, 326 arbeiten als unabhängige Sensoren auf der Leiterplatte 222. D.h. Messung und Signalaufbereitung erfolgt in den jeweiligen Sensoren 226, 326 selbst. Der Temperaturfühler 356 ist über den Leiterkamm 352 und Dickdrahbondverbindungen zwischen Leiterkamm 352 und Leiterplatte 222 an die Auswerteschaltung 228 des Luftmassenmessers 102 angebunden.

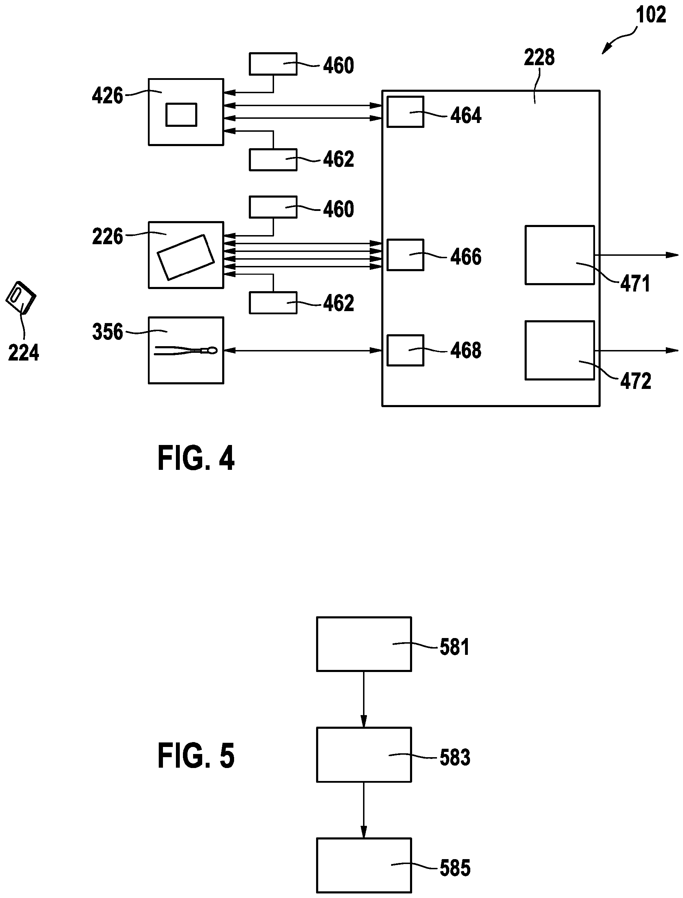

Durch die Nutzung einer digitalen SENT-Schnittstelle können alle Sensorsignale über eine Signalleitung zum Steuergerät gesendet werden. Hierzu werden die zusätzlichen Sensoren 226, 326, 356 für Druck, Temperatur und Feuchte als Satelliten an den Auswerte-IC 228 des Luftmassenmessers 102 angebunden, wie es in Fig. 4 gezeigt ist. Dieser Auswerte-IC 228 sammelt die Messdaten, bereitet sie auf und schickt sie über eine SENT-Schnittstelle beispielsweise an das Steuergerät.

Fig. 4 zeigt ein Blockschaltbild einer Luftmassenmessvorrichtung 102 gemäß einem Ausführungsbeispiel der vorliegenden Erfindung. Insbesondere ist eine Anbindung von Sensorsatelliten 226, 326, 356, wie sie beispielsweise anhand von Fig. 3 gezeigt sind, an die Auswerteschaltung 228 gezeigt.

Bei den Sensorsatelliten kann es sich, wie anhand von Fig. 3 beschrieben, um einen Feuchtigkeitssensor 426, einen Drucksensor 226 und einen Temperatursensor 356 handeln.

Gemäß diesem Ausführungsbeispiel weist der Feuchtigkeitssensor 426 einen Anschluss zu einer Versorgungsspannung (VSupply) 460, einen Anschluss zu Masse (GND) 462 und zwei bidirektionale Anschlüsse zum Verbinden des Feuchtigkeitssensors 426 mit einer ersten Eingangsschnittstelle 464 der Auswerteschaltung 228 auf. Der Feuchtigkeitssensor 426 ist gemäß diesem Ausführungsbeispiel über einen seriellen Datenbus mit der Auswerteschaltung 228 verbunden. Die erste Eingangsschnittstelle 464 ist gemäß diesem Ausführungsbeispiel als eine 12C-Schnittstelle (Inter-Integrated Circuit) ausgeführt.

Gemäß diesem Ausführungsbeispiel weist der Drucksensor 226 einen Anschluss zu einer Versorgungsspannung (VSupply) 460, einen Anschluss zu Masse (GND) 462 und vier bidirektionale Anschlüsse zum Verbinden des Drucksensors 226 mit einer zweiten Eingangsschnittstelle 466 der Auswerteschaltung 228 auf. Der Drucksensor 226 ist gemäß diesem Ausführungsbeispiel über einen synchronen seriellen Datenbus mit der Auswerteschaltung 228 verbunden. Die zweite Eingangsschnittstelle 466 ist gemäß diesem Ausführungsbeispiel als eine SPI-Schnittstelle (Serial Peripheral Interface) ausgeführt.

Gemäß diesem Ausführungsbeispiel weist der Temperatursensor 356 einen Anschluss zum Verbinden des Temperatursensors 356 mit einer dritten Eingangsschnittstelle 468 der Auswerteschaltung 228 auf. Der Temperatursensor 356 ist gemäß diesem Ausführungsbeispiel über einen analogen Anschluss mit der Auswerteschaltung 228 verbunden. Die dritte Eingangsschnittstelle 468 ist gemäß diesem Ausführungsbeispiel als Analog-Digital-Wandler ausgeführt.

Die Auswerteschaltung 228 ist gemäß diesem Ausführungsbeispiel als eine integrierte Auswerteschaltung in Form eines ASICs realisiert. Die Auswerteschaltung weist eine erste Ausgangsschnittstelle 471 und eine zweite Ausgangsschnittstelle 472 auf. Die erste Ausgangsschnittstelle 471 ist gemäß diesem Ausführungsbeispiel als eine SENT-Schnittstelle oder als eine Frequenz-Schnittstelle ausgeführt. Die zweite Ausgangsschnittstelle 472 ist gemäß diesem Ausführungsbeispiel als eine Frequenz-Schnittstelle ausgeführt.

Gemäß einem Ausführungsbeispiel ist die Auswerteschaltung 228 ausgebildet, um die über die Eingangsschnittstellen 464, 466, 468 empfangenen Sensordaten und optional zusätzlich die Sensordaten eines Luftmassensensors 224, der über eine weitere Eingangsschnittstelle mit der Auswerteschaltung 228 gekoppelt sein kann, zu kombinieren und über die als gemeinsame Schnittstelle ausgeführte erste Ausgangsschnittstelle 471 auszugeben.

Fig. 5 zeigt ein Ablaufdiagramm eines Luftmassenmessverfahrens gemäß einem Ausführungsbeispiel der vorliegenden Erfindung. Das Verfahren kann beispielsweise unter Verwendung einer Luftmassenmessvorrichtung ausgeführt werden, wie sie in den vorangegangenen Figuren gezeigt ist.

In einem Schritt 581 werden Luftmassendaten über eine erste Eingangsschnittstelle einer an einem Trägerelement angeordneten Auswerteschaltung empfangen. Die Luftmassendaten können von einem an dem Trägerelement angeordneten Luftmassensensor bereitgestellt werden.

In einem Schritt 583 werden weitere Sensordaten über zumindest eine zweite Eingangsschnittstelle der Auswerteschaltung empfangen. Die weiteren Sensordaten können von zumindest einem an dem Trägerelement angeordneten weiteren Sensor bereitgestellte werden.

In einem Schritt 585 werden gebündelte Sensordaten über eine Ausgangsschnittstelle der Auswerteschaltung ausgegeben oder bereitgestellt. Die gebündelten Sensordaten umfassen sowohl die Luftmassendaten als auch die weiteren Sensordaten. Die Luftmassendaten als auch die weiteren Sensordaten können nach dem Empfang durch die Auswerteschaltung zwischengespeichert werden, um sie in ein Übertragungsprotokoll der Ausgangsschnittstelle einbinden zu können.

## Patentansprüche

1. Luftmassenmessvorrichtung (102) für ein Fahrzeug (100), mit folgenden Merkmalen:
einem Trägerelement (220, 222);
einem Luftmassensensor (224) zum Bereitstellen von Luftmassendaten,
wobei der der Luftmassensensor (224) an dem Trägerelement (220, 222) angeordnet ist;
zumindest einem weiteren Sensor (226, 326) zum Bereitstellen weiterer Sensordaten, wobei der zumindest eine weitere Sensor (226, 326) an dem Trägerelement (220, 222) angeordnet ist; und
eine Auswerteschaltung (228) mit einer ersten Eingangsschnittstelle zum Empfangen der Luftmassendaten, zumindest einer zweiten Eingangsschnittstelle (464, 466) zum Empfangen der weiteren Sensordaten und mit einer Ausgangsschnittstelle (471), wobei die Auswerteschaltung (228) an dem Trägerelement (220, 222) angeordnet und ausgebildet ist, **dadurch gekennzeichnet, dass** die Auswerteschaltung (228) ausgebildet ist, um die Luftmassendaten und die weiteren Sensordaten entsprechend einem Übertragungsprotokoll der Ausgangsschnittstelle (471) miteinander zu kombinieren und als gebündelte Sensordaten über die Ausgangsschnittstelle (471) bereitzustellen.

2. Luftmassenmessvorrichtung (102) gemäß Anspruch 1, wobei die Ausgangsschnittstelle (471) eine Leitung oder ein Leitungspaar aufweist, über die sowohl die Luftmassendaten repräsentierende Signale als auch die weiteren Sensordaten repräsentierende Signale übertragen werden.

3. Luftmassenmessvorrichtung (102) gemäß Anspruch 1 oder 2, die als den zumindest einen weiteren Sensor (226, 326) einen Luftfeuchtesensor und/oder einen Drucksensor aufweist.

4. Luftmassenmessvorrichtung (102) gemäß einem der vorangegangenen Ansprüche, die einen Temperatursensor (256) aufweist, der über eine Sensorschnittstelle des Trägerelements (220, 222) mit dem Trägerelement (220, 222) verbunden ist.

5. Luftmassenmessvorrichtung (102) gemäß einem der vorangegangenen Ansprüche, bei der die Auswerteschaltung (228) die zweite Eingansschnittstelle (464) zum Empfangen von einem Luftfeuchtesensor (426) bereitgestellten Luftfeuchtedaten, eine dritte Eingansschnittstelle (466) zum Empfangen von einem Drucksensor (226) bereitgestellten Druckdaten und eine vierte Eingansschnittstelle (468) zum Empfangen von einem Temperatursensor (356) bereitgestellten Temperaturdaten aufweist, und bei der die Auswerteschaltung (228) ausgebildet ist, um die Luftmassendaten, die Luftfeuchtedaten, die Druckdaten und die Temperaturdaten gebündelt über die Ausgangsschnittstelle (471) bereitzustellen.

6. Luftmassenmessvorrichtung (102) gemäß einem der vorangegangenen Ansprüche, bei der die Ausgangsschnittstelle (471) der Auswerteschaltung (228) dreiadrig ausgeführt ist.

7. Luftmassenmessvorrichtung (102) gemäß einem der vorangegangenen Ansprüche, bei dem das Trägerelement (220, 222) einen Sensorträger (220) und eine Leiterplatte (222) aufweist, der Luftmassensensor (224) an dem Sensorträger (220) angeordnet ist und der zumindest eine weitere Sensor (226, 326) und die Auswerteschaltung (228) an der Leiterplatte (222) angeordnet sind.

8. Luftmassenmessvorrichtung (102) gemäß Anspruch 7, bei der die Auswerteschaltung (228) und der zumindest eine weitere Sensor (226, 326) auf derselben Seite der Leiterplatte (222) angeordnet sind.

9. Luftmassenmessvorrichtung (102) gemäß einem der vorangegangenen Ansprüche, mit einem Gehäuse (240; 340), das eine Elektronikkammer, einen Messkanal (242) zum Leiten eines Gases und einen elektrischen Anschluss (244) aufweist, wobei das Trägerelement (220, 222) in der Elektronikkammer angeordnet ist, der Luftmassensensor (224) in den Messkanal (242) eingeführt angeordnet ist und die Ausgangsschnittstelle (471) über elektrische Leitungen mit dem elektrischen Anschluss (244) verbunden ist, um die gebündelten Sensordaten an den elektrischen Anschluss (244) bereitzustellen.

10. Luftmassenmesssystem für ein Fahrzeug (100), das eine in einem Ansaugtrakt (106) eines Verbrennungsmotors (104) des Fahrzeugs (100) angeordnete oder anordenbare Luftmassenmessvorrichtung (102) gemäß Anspruch 9 aufweist, wobei die Luftmassenmessvorrichtung (102) über den elektrischen Anschluss (471) mit einem Steuergerät (110) des Fahrzeugs (100) verbunden oder verbindbar ist.

11. Luftmassenmessverfahren für ein Fahrzeug (100), das die folgenden Schritte umfasst:
Empfangen (581) von Luftmassendaten über eine erste Eingangsschnittstelle einer an einem Trägerelement (220, 222) angeordneten Auswerteschaltung (228), wobei die Luftmassendaten von einem an dem Trägerelement (220, 222) angeordneten Luftmassensensor (224) bereitgestellte Daten repräsentieren;
Empfangen (583) von weiteren Sensordaten über zumindest eine zweite Eingangsschnittstelle (464, 466) der Auswerteschaltung (228), wobei die weiteren Sensordaten von zumindest einem an dem Trägerelement (220, 222) angeordneten weiteren Sensor (226; 326) bereitgestellte Daten repräsentieren; und
Bereitstellen (585) der entsprechend einem Übertragungsprotokoll der Ausgangsschnittstelle (471) miteinander kombinierten Luftmassendaten und weiteren Sensordaten als gebündelte Sensordaten über eine Ausgangsschnittstelle (471) der Auswerteschaltung (228).

## Claims

1. Air mass measuring apparatus (102) for a vehicle (100), having the following features:
a carrier element (220, 222);
an air mass sensor (224) for providing air mass data, wherein the air mass sensor (224) is arranged on the carrier element (220, 222);
at least one further sensor (226, 326) for providing further sensor data, wherein the at least one further sensor (226, 326) is arranged on the carrier element (220, 222); and
an evaluation circuit (228) having a first input interface for receiving the air mass data, at least one second input interface (464, 466) for receiving the further sensor data, and an output interface (471), wherein the evaluation circuit (228) is arranged and formed on the carrier element (220, 222), **characterized in that** the evaluation circuit (228) is designed to combine the air mass data and the further sensor data with one another according to a transmission protocol of the output interface (471) and to provide them as bundled sensor data via the output interface (471).

2. Air mass measuring apparatus (102) according to Claim 1, wherein the output interface (471) has a line or a pair of lines, via which both signals representing the air mass data and signals representing the further sensor data are transmitted.

3. Air mass measuring apparatus (102) according to Claim 1 or 2, which has a humidity sensor and/or a pressure sensor as the at least one further sensor (226, 326).

4. Air mass measuring apparatus (102) according to one of the preceding claims, which has a temperature sensor (256) which is connected to the carrier element (220, 222) via a sensor interface of the carrier element (220, 222).

5. Air mass measuring apparatus (102) according to one of the preceding claims, in which the evaluation circuit (228) has the second input interface (464) for receiving humidity data provided by a humidity sensor (426), a third input interface (466) for receiving pressure data provided by a pressure sensor (226) and a fourth input interface (468) for receiving temperature data provided by a temperature sensor (356), and in which the evaluation circuit (228) is designed to provide the air mass data, the humidity data, the pressure data and the temperature data in a bundled manner via the output interface (471) .

6. Air mass measuring apparatus (102) according to one of the preceding claims, in which the output interface (471) of the evaluation circuit (228) has a three-wire design.

7. Air mass measuring apparatus (102) according to one of the preceding claims, in which the carrier element (220, 222) has a sensor carrier (220) and a printed circuit board (222), the air mass sensor (224) is arranged on the sensor carrier (220), and the at least one further sensor (226, 326) and the evaluation circuit (228) are arranged on the printed circuit board (222).

8. Air mass measuring apparatus (102) according to Claim 7, in which the evaluation circuit (228) and the at least one further sensor (226, 326) are arranged on the same side of the printed circuit board (222).

9. Air mass measuring apparatus (102) according to one of the preceding claims, having a housing (240; 340) which has an electronics chamber, a measuring channel (242) for conducting a gas and an electrical connection (244), wherein the carrier element (220, 222) is arranged in the electronics chamber, the air mass sensor (224) is arranged in a manner inserted into the measuring channel (242), and the output interface (471) is connected to the electrical connection (244) via electrical lines in order to provide the electrical connection (244) with the bundled sensor data.

10. Air mass measuring system for a vehicle (100), which has an air mass measuring apparatus (102) according to Claim 9 which is arranged or can be arranged in an intake tract (106) of an internal combustion engine (104) of the vehicle (100), wherein the air mass measuring apparatus (102) is connected or can be connected to a control unit (110) of the vehicle (100) via the electrical connection (471).

11. Air mass measuring method for a vehicle (100), which comprises the following steps of:
receiving (581) air mass data via a first input interface of an evaluation circuit (228) arranged on a carrier element (220, 222), wherein the air mass data represent data provided by an air mass sensor (224) arranged on the carrier element (220, 222) ;
receiving (583) further sensor data via at least one second input interface (464, 466) of the evaluation circuit (228), wherein the further sensor data represent data provided by at least one further sensor (226; 326) arranged on the carrier element (220, 222); and
providing (585) the air mass data and further sensor data combined with one another according to a transmission protocol of the output interface (471) as bundled sensor data via an output interface (471) of the evaluation circuit (228).

## Revendications

1. Dispositif de mesure de masse d'air (102), pour un véhicule (100), ayant les caractéristiques suivantes :
un élément porteur (220, 222) ;
un capteur de masse d'air (224) destiné à fournir des données de masse d'air, le capteur de masse d'air (224) étant disposé sur l'élément porteur (220, 222) ;
au moins un capteur supplémentaire (226, 326) destiné à fournir des données de capteur supplémentaires, l'au moins un capteur supplémentaire (226, 326) étant disposé sur l'élément porteur (220, 222) ; et
un circuit d'interprétation (228) comprenant une première interface d'entrée destinée à recevoir les données de masse d'air, au moins une deuxième interface d'entrée (464, 466) destinée à recevoir les données de capteur supplémentaires et comprenant une interface de sortie (471), le circuit d'interprétation (228) étant disposé et configuré sur l'élément porteur (220, 222), **caractérisé en ce que** le circuit d'interprétation (228) est configuré pour combiner entre elles les données de masse d'air et les données de capteur supplémentaires conformément à un protocole de transmission de l'interface de sortie (471) et à les fournir par le biais de l'interface de sortie (471) sous la forme de données de capteur combinées.

2. Dispositif de mesure de masse d'air (102) selon la revendication 1, l'interface de sortie (471) possédant une ligne ou une paire de lignes par le biais de laquelle sont transmis à la fois les signaux représentant les données de masse d'air et les signaux représentant les données de capteur supplémentaires.

3. Dispositif de mesure de masse d'air (102) selon la revendication 1 ou 2, qui possède en tant qu'au moins un capteur supplémentaire (226, 326) un capteur d'humidité de l'air et/ou un capteur de pression.

4. Dispositif de mesure de masse d'air (102) selon l'une des revendications précédentes, qui possède un capteur de température (256), lequel est relié à l'élément porteur (220, 222) par le biais d'une interface de capteur de l'élément porteur (220, 222).

5. Dispositif de mesure de masse d'air (102) selon l'une des revendications précédentes, avec lequel le circuit d'interprétation (228) possède la deuxième interface d'entrée (464) destinée à recevoir des donnés d'humidité de l'air fournies par un capteur d'humidité de l'air (426), une troisième interface d'entrée (466) destinée à recevoir des données de pression fournies par un capteur de pression (226) et une quatrième interface d'entrée (468) destinée à recevoir des données de température fournies par un capteur de température (356), et avec lequel le circuit d'interprétation (228) est configuré pour fournir les données de masse d'air, les donnés d'humidité de l'air, les données de pression et les données de température combinées par le biais de l'interface de sortie (471).

6. Dispositif de mesure de masse d'air (102) selon l'une des revendications précédentes, avec lequel l'interface de sortie (471) du circuit d'interprétation (228) est configurée à trois fils.

7. Dispositif de mesure de masse d'air (102) selon l'une des revendications précédentes, avec lequel l'élément porteur (220, 222) possède un porte-capteur (220) et un circuit imprimé (222), le capteur de masse d'air (224) est disposé sur le porte-capteur (220) et l'au moins un capteur supplémentaire (226, 326) ainsi que le circuit d'interprétation (228) sont disposés sur le circuit imprimé (222).

8. Dispositif de mesure de masse d'air (102) selon la revendication 7, avec lequel le circuit d'interprétation (228) et l'au moins un capteur supplémentaire (226, 326) sont disposés sur le même côté du circuit imprimé (222).

9. Dispositif de mesure de masse d'air (102) selon l'une des revendications précédentes, comprenant un boîtier (240 ; 340) qui possède une chambre électronique, un canal de mesure (242) destiné à conduire un gaz et une borne électrique (244), l'élément porteur (220, 222) étant disposé dans la chambre électronique, le capteur de masse d'air (224) étant disposé introduit dans le canal de mesure (242) et l'interface de sortie (471) étant reliée à la borne électrique (244) par le biais de lignes électriques afin de fournir les données de capteur combinées au niveau de la borne électrique (244).

10. Système de mesure de masse d'air pour un véhicule (100), lequel possède un dispositif de mesure de masse d'air (102) selon la revendication 9 disposé ou pouvant être disposé dans un conduit d'admission (106) d'un moteur à combustion interne (104) du véhicule (100), le dispositif de mesure de masse d'air (102) étant relié ou pouvant être relié à un contrôleur (110) du véhicule (100) par le biais de la borne électrique (471).

11. Procédé de mesure de masse d'air (100), pour un véhicule (100), comprenant les étapes suivantes :
réception (581) de données de masse d'air par le biais d'une première interface d'entrée d'un circuit d'interprétation (228) disposé sur un élément porteur (220, 222), les données de masse d'air représentant des données fournies par un capteur de masse d'air (224) disposé sur l'élément porteur (220, 222) ;
réception (583) de données de capteur supplémentaires par le biais d'au moins une deuxième interface d'entrée (464, 466) du circuit d'interprétation (228), les données de capteur supplémentaires représentant des données fournies par au moins un capteur supplémentaire (226, 326) disposé sur l'élément porteur (220, 222) ; et
fourniture (585) des données de masse d'air et des données de capteur supplémentaires combinées entre elles conformément à un protocole de transmission de l'interface de sortie (471) sous la forme de données de capteur combinées par le biais d'une interface de sortie (471) du circuit d'interprétation (228).
